(19) Europäisches Patentamt
European Patent Office
Office européen des brevets

(11) EP 2 359 803 B1

(12) EUROPEAN PATENT SPECIFICATION

(45) Date of publication and mention of the grant of the patent:
**11.06.2014 Bulletin 2014/24**

(51) Int Cl.:
***A61K 8/37*** *(2006.01)* ***A61Q 5/12*** *(2006.01)*
***A61Q 9/02*** *(2006.01)* ***A61Q 19/10*** *(2006.01)*

(21) Application number: **10153434.5**

(22) Date of filing: **12.02.2010**

(54) **Cosmetic composition containing polyglycerol partial ester**

Kosmetikzusammensetzung mit Polyglycerolpartialester

Composition cosmétique contenant un ester partiel de polyglycérol

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(43) Date of publication of application:
**24.08.2011 Bulletin 2011/34**

(73) Proprietor: **Evonik Degussa GmbH**
**45128 Essen (DE)**

(72) Inventors:
- **Wenk, Hans Henning**
**45470, Mülheim an der Ruhr (DE)**
- **Lersch, Peter**
**46537, Dinslaken (DE)**

(56) References cited:
**EP-A1- 1 454 612** **EP-A1- 1 857 091**
**EP-A1- 1 857 092**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

Field of the invention

The present invention relates to cosmetic compositions containing polyglycerol partial esters.

Background of the invention

The use of polyglycerol esters in cosmetic applications is known *per se*:

German Patent Publication DE 38 18 292 A1 relates to a process for the preparation of fatty acid or hydroxy fatty acid esters of isopropylidene derivatives of a polyglycerol and their use for cosmetic preparations and skin care agents.

European Publication EP 0 451 461 B1 describes the use of mixtures of polyglycerol fatty acid esters as emulsifiers in cosmetic and pharmaceutical preparations. These are obtainable by partial esterification of polyglycerols with at least one saturated fatty acid having 12 to 22 C atoms or at least one unsaturated fatty acid having 16 to 22 C atoms, where the unsaturated fatty acid or fatty acid mixture employed can additionally contain up to 10% by weight of saturated fatty acids having 16 to 22 C atoms. The degree of esterification of the saturated or unsaturated fatty acids in the mixture is between 20 and 70%.

European Publication EP 0 451 461 B1 describes the use of mixtures of polyglycerol fatty acid esters as emulsifiers in cosmetic and pharmaceutical preparations. These are obtainable by partial esterification of polyglycerols with at least one saturated fatty acid having 12 to 22 C atoms or at least one unsaturated fatty acid having 16 to 22 C atoms, where the unsaturated fatty acid or fatty acid mixture employed can additionally contain up to 10% by weight of saturated fatty acids having 16 to 22 C atoms. The degree of esterification of the saturated or unsaturated fatty acids in the mixture is between 20 and 70%.

JP2008308415 describes hair rinses, hair treatments, or hair conditioners which suppress increased volume of hair in high moisture environments and contain methacrylate polymers, fatty acid polyglycerin esters, alditols, and cationic surfactants. Diglycerol diisostearate is given as a suitable example of a polyglycerol ester.

JP2008280329 describes polyglycerin fatty acid esters as gelling agents for cosmetic oils.

JP2008208050 describes detergents containing polyalkyl glucoside derivatives and polyglycerin monofatty acid esters (monoesterification degree >= 70%) having excellent foaming properties and good skin compatibility. JP2008195689 describes skin and hair care compositions comprising microemulsions and containing polyglycerin esters with C8-22 fatty acids and having an HLB value >=13. W02005115328 discloses skin and hair care products having an improved performance profile containing cationic polymers and polyglycerin fatty acid esters.

Mashiko et al. describe the use of polyglycerin isostearates in hair care products (Fragrance Journal 1998, 26(5), 64-70).

Takano et al. describe the use of Nikkomulese 61H (containing polyglyceryl-10 pentastarate) for hair conditioner applications.

EP780117 discloses hair conditioning emulsions containing a C6-22 fatty acid ester with polyglycerol.

DE3533600 discloses hair preparations containing water soluble, polyglycerol containing non-ionic surfactants.

It was an object of the invention to provide agents which overcome at least one disadvantage listed in the prior art.

It has now been found, surprisingly, that cosmetic compositions containing polyglycerol partial esters of claim 1 fulfill the requirements.

The present invention therefore relates to cosmetic compositions containing polyglycerol partial ester having the structure of Formula (I)

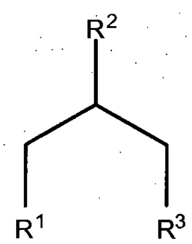

Formula (I)

with $R^1$, $R^2$ and $R^3$ independent from each other, equal or different selected from the group consisting of

-OH,
$-OR^4$, with $R^4$ a linear, unsubstituted acyl radical with a chain length of from 16 to 22 carbon atoms with the proviso that the monocarboxylic acids obtained from the acyl radical by saponification bears an iodine value of smaller than 50,
a radical having the structure of general Formula (II)

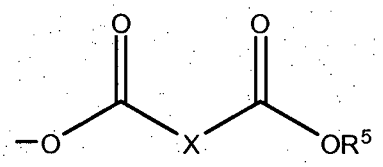
Formula (II)

with $R^5$ a radical having the structure of Formula (I) wherein one of $R^1$, $R^2$ und $R^3$ being a direct bond to the oxygen of $-OR^5$ and with X a bivalent organic residue with from 2 to 34 carbon atoms and
$-OR^5$, with $R^5$ like above

wherein each molecule of the polyglycerol partial ester comprises at least one of each $-OR^5$ and a radical having the structure of Formula (II),
with the provisos that the polyglycerol partial ester comprises an HLB-value from 2 to 10 and
that the polyglycerol obtained by hydrolysis or alcoholysis of the polyglycerol partial ester comprises an average degree of polymerization of from 2 to 8 and at least 1 % of the polyglycerol comprises cyclic structures.

The invention further relates to the use of the polyglycerol partial esters in cosmetic preparations.

An advantage of the present invention is that the cosmetic preparations according to the invention are long time storage stable.

A further advantage of the present invention is that the cosmetic preparations comprising the polyglycerol partial esters are stable at high temperature and also withstand repeated freeze-thaw-cycles.

Yet a further advantage is that the cosmetic preparations according to the invention might give a light, non-greasy skin feel.

Yet a further advantage of the present invention is that the cosmetic preparations might enhance the combability of hair.

Yet a further advantage of the present invention is that the cosmetic preparations might enhance the grip of hair. Yet a further advantage of the present invention is that the cosmetic preparations might enhance the look of hair. Yet a further advantage of the present invention is that the cosmetic preparations might enhance the elasticity of hair.

Yet a further advantage of the present invention is that the cosmetic preparations may protect human or animal hair against heat damage.

Still another advantage of the present invention is that the polyglycerol partial esters may need little deposition aid to settle on fibers.

Yet a further advantage of the present invention is that the cosmetic preparations according to the invention may have an enhanced deposition of the polyglycerol partial ester in the presence of an anionic surfactant.

Another advantage is that the cosmetic compositions according to the invention may provide excellent static control on fibers.

A further advantage is that the polyglycerol partial esters used in the invention may have excellent emulsifying properties.

Another advantage is that the cosmetic compositions according to the invention may be biodegradable and may have a low human and environmental toxicity.

Yet another advantage is that the cosmetic compositions according to the invention may allow for formulation with material that are not stable at low pH such as enzymes and certain perfumes.

The person skilled in the art will acknowledge that polyglycerol esters due to their polymeric nature and due to the methods they are prepared by are statistical mixtures of different structures.

Thus, a polyglycerol molecule may comprise ether bonds between two primary positions, a primary and a secondary position, or two secondary positions of the glycerol monomer units. Cyclic structures comprising one or more cycles may also be present. For tetraglycerol and higher oligomers, branched structures comprising at least one glycerol monomer unit linked to three further glycerol monomer units via an ether linkage may be present. A polyglycerol mixture may contain different oligomers and isomers of these, and may be characterized by the oligomer distribution, i. e. the proportion of mono-, di-, tri-, ...-glycerol structures in the mixture. This distribution can for example be determined by

high temperature gas chromatography of the polyglycerol mixture after derivatization. Synthesis of single oligoglycerol isomers is described in "Original synthesis of linear, branched and cyclic oligoglycerol standards", Cassel et al., Eur. J. Org. Chem. 2001, 875-896.

Additionally, the esterification of polyglycerol mixtures typically results in a distribution of non-esterified polyglycerol, monoester, diester, triester, etc., where the average degree of esterification is determined by the ratio of fatty acid (or its derivative) to polyglycerol used in the synthesis. If a mixture of different fatty acids is used for the esterification, more than one equal or different fatty acid residues may be linked to one polyglycerol molecule via ester linkage.

Additional esterification with dicarboxylic acids results in oligomerization or cross-linking of the polyglycerol ester substructures. The oligomers or polymers thus formed may be linear polyesters of the general structure $A(BA)_n$ or $B(AB)_n$, where A is a polyglycerol or polyglycerol fatty acid ester moiety, and B is a dicarboxylic acid moiety, but may also comprise branched or multi-branched structures. The average degree of polymerization of the polyester is determined by the ratio or polyglycerol fatty acid ester and dicarboxylic acid (or its derivative) in the esterification process.

For the present invention it is essential that the hydrophilic-lipophilic balance value (HLB value) of the polyglycerol partial ester is between 2 and 10. The HLB value is a measure of the degree to which the molecule is hydrophilic or lipophilic, determined by calculating values for the different regions of the molecule. For the purpose of the present invention, the HLB value of the polyglycerol partial esters is calculated as follows:

$$HLB = (mp/(mp+ma))*20,$$

where mp is the mass of polyglycerol, and ma is the mass of carboxylic acid mixture (comprising mono- and dicarboxylic acid) used in the synthesis of the polyglycerol ester. For example, esterification of 100 g polyglycerol with 90 g monocarboxylic acid and 10 g dicarboxylic acid would result in an HLB of (100 g / (90 g + 10 g + 100 g) ) *20 = 10, independent of the degree of polymerization of the polyglycerol and the type of carboxylic acids used.

For the present invention it is essential that the polyglycerol backbone of the polyglycerol partial ester comprises an average degree of polymerization of from 2 to 8, preferred from 2.5 to 6, particularly preferred from 3 to 4.5.

A suitable method for determining the oligomer distribution of the polyglycerol in a given polyglycerol partial ester comprises hydrolysis or alcoholysis of the partial ester, separation of the resulting polyglycerol from the formed carboxylic acid compounds, and analysis by gas chromatography after derivatization.

For this purpose, the 0.6 g polyglycerol ester is refluxed in 25 ml of 0.5 N ethanolic KOH for 30 minutes and adjusted to pH 2-3 with sulphuric acid. The fatty acids are separated by threefold extraction with an equivalent volume of petroleum ether. The combined extracts are evaporated to a volume of approx. 10 ml. A 0.5 ml aliquot is transferred to an autosampler vial and analyzed by GC after addition of 0.5 ml MTBE and 1 ml TMPAH solution (trimethylanilinium hydroxide in mehanol) as derivatization agent.

Fatty acid GC-analysis is carried out with a gas-chromatograph equipped with split/splitless injector, capillary column and a flame ionisation detector.

| | | |
|---|---|---|
| Conditions: | | |
| Injector: | 290 °C,Split 30 ml | |
| Injection volume: | 1 μl | |
| Column: | 30 m *0.32 mm HP1 0.25 μm | |
| Carrier gas: | helium, head pressure 70 kPa | |
| Temp. prog. : (conditioning) | 80 °C - 300 °C with 8 °C/min; | |
| Detector: | FID at | 320 °C |
| | hydrogen | 35 ml/min |
| | air | 240 ml/min |
| | make up gas | 35 ml/min |

Applying these conditions the fatty acids methyl esters are separated according to their alkyl chain length.

The relative content of the individual fatty acids (chain length distribution) is evaluated by peak area percentage. The residue after extraction with petroleum ether is adjusted to pH 7-8 by addition of barium hydroxide solution. The precipitate of barium sulphate is separated by centrifugation. The supernatant is removed and the residue extracted thrice with 20 ml of ethanol. The combined supernatants are evaporated at 80 °C / 50 mbar. The residue is dissolved in pyridine. 500 μl of the solution are transferred to an autosampler vial and 1 ml of MSTFA (N-Methyl-N-trifluoroacetamide)

is added. The vial is closed and heated to 80 °C for 30 minutes.

GC-analysis of the polyglycerol component (as its trimethylsilyl derivative) is carried out with a gas-liquid chromatograph equipped with a on column injector and FID detector.

| Conditions: | |
|---|---|
| Injector: | on column, oven tray |
| Injection volume: | 0.1 μl |
| Carrier gas: | 3 ml/min Hydrogen (constant flow) |
| Column (Varian) | SimDist 12 m x 0.32 mm x 0.1 μm |
| Temperature program: | 65 °C - 365 °C,10 °C/min |
| Detector (FID): | 375 °C |

Under these conditions, polyglycerols are separated according to their degree of polymerization. Additionally, cyclic isomers are separated from linear ones up to a degree of polymerization of four.

The peak areas of the individual oligomers are separated by a perpendicular applied at the lowest point of the peak valley in between.

Since the resolution of oligomers higher than hexaglycerol is poor, peaks of heptaglycerol and higher oligomers are summarized as "heptaglycerol and higher" and treated as heptaglycerol for the purpose of polydispersity index calculation. Also, for the calculation of the polydispersity index linear and cyclic isomers are summarized.

The relative ratio of the individual polyglycerol oligomers and isomers is calculated from the peak area of the GC obtained as described.

Of course, the described GC analyses of the fatty acid component and polyglycerol component can also be performed on the raw materials which had been used for the preparation of the polyglycerol esters according to the invention.

Polyglycerol depending on its way of preparation can comprise different percentages of cyclic structures. An overview of some cyclic structures present in commercial polyglycerol mixtures is given in "Original synthesis of linear, branched and cyclic oligoglycerol standards", Cassel et al., Eur. J. Org. Chem. 2001, 875-896. For the polyglycerol partial esters it is essential, that the polyglycerol in the polyglycerol backbone of the partial ester comprises at least 1 %, preferably at least 2 % and even more preferred at least 3 % cyclic structures.

The given percentages are neither percentages by weight nor per mole but are determined by the GC method described above and base on the amount of all polyglycerol.

It is advantageous if the polyglycerol partial esters comprise a polyglycerol backbone in that the polyglycerol comprises a polydispersity index of greater than 0.6, preferably greater than 1.0, particularly preferably greater than 1.2.

For the purpose of the present invention, the polydispersity index is calculated as

$$\sum_i |n_i - <n>| \cdot x_i \ ,$$

where $n_i$ is the degree of polymerization of the single oligomer i, <n> is the average degree of polymerization of the polyglycerol mixture, and $x_i$ is the proportion of the oligomer i in the polyglycerol mixture as determined by the GC method described above. For this calculation, the average degree of polymerization <n> is calculated from the hydroxyl value (OHV, in mg KOH/g) according to the formula <n> = (112200 - 18*OHV) / (74*OHV - 56100).

The radicals $R^5$ in the polyglycerol partial ester might be the same or different within one molecule, preferably they are different.

It is obvious, that the residue $-OR^4$ is determined by the monocarboxylic acid $HOR^4$ used in the esterification reaction for preparing the polyglycerol partial ester. Preferred residues $-OR^4$ are accordingly derived from the acids selected from the group consisting of palmitic acid, stearic acid, arachidic acid, and behenic acid. Mixtures of different acids can be used, too, especially technical mixtures like for example fully or partially hydrogenated palm fatty acids, palm kernel fatty acids, coconut fatty acids, soybean fatty acids, tallow fatty acids, rapeseed fatty acids, high erucic rapeseed fatty acids or distilled fractions of these as long as their iodine value is smaller than 50, preferred smaller than 30 and more preferred smaller than 25. Depending on the degree of hydrogenation and the raw material, these technical mixtures can contain certain amounts of unsaturated fatty acids which then are contained in the polyglycerol partial ester according to the invention. Typical examples of these unsaturated fatty acids are palmitoleic acid, oleic acid, elaidic acid, erucic acid, linoleic acid, and linolenic acid, where oleic acid and elaidic acid are most commonly found as constituents

of partially hydrogenated fatty acid mixtures. The amount of this byproduct can be determined by the iodine value of the fatty acids obtained from the acyl radical by saponification of the polyglycerol partial ester. It is essential to the polyglycerol partial ester of the present invention, that this iodine value is smaller than 50, more preferred smaller than 30 and even more preferred from 1 to 25.

The iodine value can be determined by DIN 53241-1:1995-05.

It is also obvious, that the radical having the structure of general Formula (II) is derived from a dicarboxylic acid having the structure of general Formula (IIb).

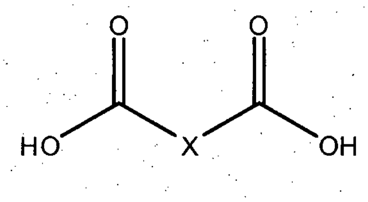

Formula (IIb)

In a preferred embodiment the radical of Formula (II) is derived from the group of dicarboxylic acids known as dimer fatty acids. The dimer fatty acids employed are a mix of acyclic and cyclic dicarboxylic acids which are obtained by a catalysed dimerization of unsaturated fatty acids having 12 to 22 C atoms and have an average functionality of 2 to 3, preferably approximately 2. They can also comprise polymeric fatty acids (trimeric and of higher functionality) in a minor amount. The acid numbers are in the range from 150 to 290, preferably 190 to 200. Commercially available products have on average monomer contents of approximately 7 to 15 wt. %, dimer contents of approximately 70 to 77 wt. % and polymer contents of approximately 15 to 16 wt. %. They can be adjusted to higher contents of the particular functionalities (mono, di, tri) by known separation processes and/or to low contents of unsaturated fatty acids (low iodine numbers) by hydrogenation. Further information is to be found in the products sheets of the manufacturers, such as, for example, of Croda/Uniqema (Pripol®) and Arizona Chem. (Unidyme®, Century.RTM.). For the preparation and use of dimer acids and the physical and chemical properties thereof, reference is also made to the publication "The Dimer Acids: The chemical and physical properties, reactions and applications", ed. E. C. Leonard; Humko Sheffield Chemical, 1975, Memphis, Tenn.

The use of relatively short-chain dicarboxylic acids instead of dimer acids, such as succinic acid, maleic acid, fumaric acid, glutaric acid, adipic acid, pimelic acid, suberic acid, azelaic acid, sebacic acid and dodecanedioic acid, is particularly suitable for the intended use according to the invention as cosmetic ingredient. Hydroxydicarboxylic acids, such as malic acid and tartaric acid are also suitable.

Also suitable are aromatic dicarboxylic acid, in particular phthalic acid, isophthtalic acid, and terephthalic acid. The alkanedicarboxylic acids having 4 to 14 C atoms are particularly preferred.

Preferred cosmetic compositions therefore contain polyglycerol partial esters in that X is a bivalent, linear, unsubstituted alkyl radical with 2 to 12 carbon atoms.

Also preferred according to the invention therefore contain polyglycerol partial esters which have a molecular weight (number averaged molecular weight Mn by gel permeation chromatography) of at least 2000 g/mol, preferably at least 2400 g/mol, more preferably at least 2700 g/mol. Obviously, the molecular weight of the polyglycerol ester can be increased by increasing degree of polymerization of the polyglycerol, degree of esterification with monocarboxylic acid(s), degree of esterification with dicarboxylic acid(s), or more than one of these parameters at the same time.

Preferably cosmetic compositions according to the invention contain polyglycerol partial esters in that the molar ratio of the monocarboxylic acid component to the dicarboxylic acid component is 2-20, preferably 2.5-10, more preferably 3-8.

Preferably cosmetic compositions according to the invention contain polyglycerol partial esters in that the molar ratio of the dicarboxylic acid component to polyglycerol is in the range of from 0.1 to 1.0, preferably from 0.15 to 0.9, more preferably from 0.3 to 0.8.

Preferably cosmetic compositions according to the invention contain polyglycerol partial esters having a melting point of at least 25 °C, preferably of at least 35 °C, more preferably of at least 40 °C.

The partial esters contained in the cosmetic composition according to the present invention are obtainable by a process of esterification of

a) a polyglycerol mixture comprising an average degree of polymerization of from 2 to 8, preferred from 2.5 to 6, particularly preferred from 3 to 4.5, and at least 1 % of cyclic structures, with

b) at least one monocarboxylic acid comprising a carboxylic acid $HOR^4$, with $R^4$ a linear, unsubstituted acyl radical with a chain length of from 16 to 22 carbon atoms with the proviso that the the carboxylic acid or mixture of carboxylic acids bears an iodine value of smaller than 50, preferably smaller than 30, particularly preferably smaller than 25,

c) at least one dicarboxylic acid having the structure of general Formula (IIb).

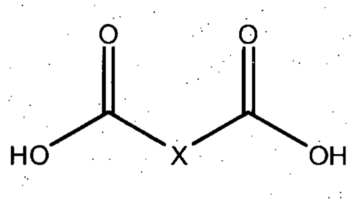 Formula (IIb)

with X a bivalent organic residue with from 2 to 34 carbon atoms
or mixtures thereof
with the provisio that the ratio by weight of polyglycerol mixture to the sum of monocarboxylic acid and dicarboxylic acid is in the range from about 0.11 to 1, preferably in the range from about 0.11 to 0.67.

The iodine value and the mean degree of polymerization can be determines as described above.

It is obvious that instead of the monocarboxylic acids b) and the dicarboxylic acids c) suitable derivatives of the carboxylic acids like their anhydrides, their halogenides, and their esters, preferably their esters with short chain alcohols like methanol or ethanol, may be used to obtain the polyglycerol esters contained in the cosmetic composition according to the invention.

The process of preparing polyglycerol partial ester can be in two stages which proceed in a manner known *per se.* First in step 1.) the polyglycerol is esterified with the at least one carboxylic acid, in a second step 2.) the at least one dicarboxylic acid is added.

It may be beneficial to apply a catalyst (e. g. hydroxides or carbonates of alkali metals; hydroxides of alkaline earth metals; sulfonic acid catalysts like p-toluenesulfonic acid, methanesulfonic acid, trifluoromethanesulfonic acid; metal oxides like zinc(II)oxide or tin(II)oxide) in the esterification process, however the reaction may be performed without addition of a catalyst. The esterification reaction is typically performed at temperatures between 160 and 270 °C, preferably between 180 and 250 °C. A suitable pressure range for the reaction is from about 50 mbar to about 1200 mbar, preferably from about 600 mbar to ambient pressure. The lower applicable pressure is limited by loss of carboxylic acids from the reaction mixture by distillation. Preferred polyglycerols used in the process for preparing the polyglycerol partial ester contained in the cosmetic composition according to the present invention comprise an average degree of polymerization of 2-8, preferably 2.5-6, particularly preferably 3-4.5.

The polyglycerol used in the esterification process described above can be produced by several methods. Suitable methods for the production of polyglycerol include polymerization of glycidol (e. g. with base catalysis), polymerization of epichlorohydrin (e. g. in the presence of equimolar amounts of a base like NaOH), or polycondensation of glycerol.

The preferred method for the purpose of this invention is condensation of glycerol, in particular in the presence of catalytic amounts of base, preferably NaOHor KOH. Suitable reaction conditions include temperatures of 220-260 °C and reduced pressure (20-800 mbar, preferably 50-500 mbar) to facilitate removal of reaction water from the mixture. The progress of the condensation reaction may be followed by measuring refractive index, viscosity, or hydroxyl value of the reaction product.

A particularly preferred method, which results in a desired broader polydispersity of the product, comprises the steps of

- reacting glycerol in a condensation reaction in the presence of a catalytic amount (0.2-5% by weight) of base at a temperature from about 220-260 °C at a pressure between 250 and 1000 mbar while removing reaction water by distillation until the reaction mixture contains less than 70% (preferably less than 60%) of glycerol
- continuing the condensation reaction at a lower pressure between 20 and 200 mbar while removing reaction water and glycerol by distillation until the hydroxyl value of the reaction mixture is lower than 1400 (preferably lower than 1200), and
- optionally neutralizing the catalyst with an acid.

In the method for preparing the polyglycerol partial ester contained in the cosmetic composition according to the present invention preferred monocarboxylic acids used are selected from the group consisting of palmitic acid, stearic acid, arachidic acid, and behenic acid. Mixtures of different acids can be used, too, especially technical mixtures like the technical mixtures of fatty acids mentioned above which may contain some amounts of unsaturated fatty acids.

Preferred dicarboxylic acids of general Formula (IIb) in the method for preparing the polyglycerol partial ester contained in the cosmetic composition according to the present invention are the above named dimer acids and relatively short-chain dicarboxylic acids, such as succinic acid, maleic acid, fumaric acid, glutaric acid, adipic acid, pimelic acid,

suberic acid, azelaic acid, sebacic acid and dodecanedioic acid. Hydroxydicarboxylic acids, such as malic acid and tartaric acid are also suitable. Also suitable are aromatic dicarboxylic acid, in particular phthalic acid, isophthtalic acid, and terephthalic acid. The alkanedicarboxylic acids having 4 to 14 C atoms are particularly preferred to be used in the method for preparing the polyglycerol partial ester contained in the cosmetic composition according to the present invention.

In a preferred method for preparing the polyglycerol partial ester contained in the cosmetic composition according to the present invention the molar ratio of component b) to component c) is 2-20, preferably 2.5-10, more preferably 3-8.

Furthermore a preferred method for preparing the polyglycerol partial ester contained in the cosmetic composition according to the present invention is characterized in that the molar ratio of the dicarboxylic acid component to polyglycerol is 0.1-1.0, preferably 0.15-0.9, more preferably 0.3-0.8.

Preferred cosmetic compositions according to the invention contain the aforementioned polygylcerol partial esters in an amount from 0.1 wt.% to 10.0 wt.%, more preferably from 0.15 wt.% to 5.0 wt.%, even more preferred from 0.2 wt.% to 4.0 wt.% based on the total weight of the composition.

Preferred cosmetic compositions according to the invention are emulsions, preferably oil in water or water in oil emulsions or multiple emulsions in the form of lotions, creams, sprays or microemulsions.

The cosmetic composition according to the invention can, for example, comprise at least one additional component selected from the group of
emollients,
emulsifiers and surfactants,
thickeners/viscosity regulators/stabilizers,
UV photoprotective filters,
antioxidants and vitamins,
hydrotropes (or polyols),
solids and fillers,
film formers,
pearlescent additives,
deodorant and antiperspirant active ingredients,
insect repellents,
self-tanning agents,
preservatives,
conditioners,
perfumes,
dyes,
biogenic active ingredients,
care additives,
superfatting agents and
solvents.

Substances to be used as additional components are well known to the artesan, for further exemplary substances the list in for example DE 102008001788 can be consulted.

Preferred cosmetic compositions according to the invention contain at least one additional component selected from cationic surfactants and polymers containing at least one quaternary ammonium group. Preferred cationic surfactants are selected from quaternary ammonium salts containing organic carbon chains like for example cetrimonium chloride, behentrimonium chloride, dicetyldimonium chloride, quaternium-18, behentrimonium methosulfate, distearoylethyl dimonium chloride, palmitamidopropyltrimonium chloride, ricinoleamidopropyltrimonium methosulfate, distearyldimonium chloride and quaternium-87.

Preferred cosmetic compositions according to the invention therefore may contain
0 wt.% to 10 wt.%, preferably von 0.1 wt.% bis 7.5 wt.% of at least one emulsifier,
0 wt.% to 10 wt.%, preferably von 0.1 wt.% bis 7.5 wt.% of at least one consistency enhancer,
0.1 wt.% to 10 wt.%, preferably von 0.1 wt.% bis 7.5 wt.% of at least one cationic surfactant and/or at least one polymer containing at least one quaternary ammonium group 0 wt.% to 20 wt.%, preferably von 0.1 wt.% bis 17.5 wt.% of at least one cosmetic oil or emollient, wherein all percentages base on the total weight of the composition.

Preferably, the cosmetic compositions are cleaning and care compositions.

Cleaning and care compositions are understood as meaning primarily those compositions for the treatment of hair or skin, in particular hair. Such hair care compositions are, for example, hair shampoos, liquid soaps, hair rinses, permanent wave neutralizing lotions, hair colour shampoos, hair setting compositions, hair arranging compositions, hair styling preparations, blow-drying lotions, foam setting compositions, hair treatments, leave-in conditioners and other cleaning and care formulations.

Another part of the invention is the use of the above described polyglycerol partial esters contained in the

cosmetic compositions according to the invention in cosmetic compositions, especially in hair care compositions, wherein polyglycerol partial esters contained in preferred cosmetic compositions according to the invention are particularly preferably used.

Another part of the invention is the use of the above described polyglycerol partial esters contained in the cosmetic compositions according to the invention as a conditioning agent for hair, wherein polyglycerol partial esters contained in preferred cosmetic compositions according to the invention are particularly preferably used.

Another part of the invention is the use of the above described polyglycerol partial esters contained in the cosmetic compositions according to the invention as a hair protecting agent, especially in protection from heat, wherein polyglycerol partial esters contained in preferred cosmetic compositions according to the invention are particularly preferably used.

Another part of the invention is the use of the above described polyglycerol partial esters contained in the cosmetic compositions according to the invention as a hair repair agent, especially in repairing thin and fine hair, wherein polyglycerol partial esters contained in preferred cosmetic compositions according to the invention are particularly preferably used.

Another part of the invention is the use of the above described polyglycerol partial esters contained in the cosmetic compositions according to the invention as a hair strengthening agent, especially in strengthening thin and fine hair, wherein polyglycerol partial esters contained in preferred cosmetic compositions according to the invention are particularly preferably used.

Yet another part of the invention is the use of the cosmetic compositions according to the invention in the areas described in each of the uses for the polyglycerol partial esters above, wherein preferred cosmetic compositions according to the invention are particularly preferably used.

Examples:

*Example 1: Preparation of [PGE 37]*

500 g glycerol and 2.5 g of potassium hydroxide were heated to 240 °C at a pressure of 400 mbar while nitrogen was bubbled through the mixture. Reaction water was continuously distilled from the reaction mixture. When the refractive index reached 1.4920, the reaction mixture was cooled and subjected to thin film distillation at a temperature of 250 °C and a pressure of 4 mbar.
The distillation residue had a hydroxyl value of 1150 mg KOH/g, a polydispersity index of 0.71 and contained 1.5 % of cyclic polyglycerols
215.1 g of this product were reacted with 494.3 g of partially hydrogenated tallow fatty acid (C16/18) with an iodine value of 20 at a temperature of 240 °C while sparging with nitrogen. Reaction water was continuously distilled from the mixture. When the acid value reached <10 mg KOH/g, 90.6 g sebacic acid were added to the reaction mixture and the reaction was continued to an acid value of
<3 mg KOH/g. Subsequently, the reaction was stopped by cooling.
Hydroxyl value: 112 mg KOH/g
Acid value: 2.1mg KOH/g
Saponification value: 201 mg KOH/g
HLB (calculated): 5.4
Molar ratio monoacid:diacid: 4.0

*Example 2: Preparation of* [PGE 38]

500 g glycerol and 2.5 g of potassium hydroxide were heated to 240 °C at a pressure of 400 mbar while nitrogen was bubbled through the mixture. Reaction water was continuously distilled from the reaction mixture. When the refractive index reached 1.4920, the reaction mixture was cooled and subjected to thin film distillation at a temperature of 250 °C and a pressure of 4 mbar.

The distillation residue had a hydroxyl value of 1150 mg KOH/g, a polydispersity index of 0.71 and contained 1.5 % of cyclic polyglycerols
208 g of this product were reacted with 478.1 g of partially hydrogenated tallow fatty acid (C16/18) with an iodine value of 20 at a temperature of 240 °C while sparging with nitrogen. Reaction water was continuously distilled from the mixture. When the acid value reached <10 mg KOH/g, 113.9 g sebacic acid were added to the reaction mixture and the reaction was continued to an acid value below 3 mg KOH/g. Subsequently, the reaction was stopped by cooling.
Hydroxyl value: 91 mg KOH/g
Acid value: 1.7 mg KOH/g
Saponification value: 219 mg KOH/g

HLB (calculated): 5.2
Molar ratio monoacid:diacid: 3.1

*Example 3: Preparation of* [PGE 39]

500 g glycerol and 2.5 g of potassium hydroxide were heated to 240 °C at a pressure of 400 mbar while nitrogen was bubbled through the mixture. Reaction water was continuously distilled from the reaction mixture. When the refractive index reached 1.4920, the reaction mixture was cooled and subjected to thin film distillation at a temperature of 250 °C and a pressure of 4 mbar.
The distillation residue had a hydroxyl value of 1150 mg KOH/g, a polydispersity index of 0.71 and contained 1.5 % of cyclic polyglycerols.
204.3 g of this product were reacted with 352.1 g of partially hydrogenated tallow fatty acid (C16/18) with an iodine value of 20 at a temperature of 240 °C while sparging with nitrogen. Reaction water was continuously distilled from the mixture. When the acid value reached <10 mg KOH/g, 243.6 g Dimer acid (Radiacid 0977, Oleon) were added to the reaction mixture and the reaction was continued to an acid value of <3 mg KOH/g. Subsequently, the reaction was stopped by cooling.
Hydroxyl value: 121 mg KOH/g
Acid value: 0.8 mg KOH/g
Saponification value: 158 mg KOH/g
HLB (calculated): 5.1
Molar ratio monoacid:diacid: 3.0
Average DP of polyglycerol: 3.2 (OHV 1150)

*Example 4: Preparation of* [PGE 58]

500 g glycerol and 2.5 g of potassium hydroxide were heated to 240 °C at a pressure of 400 mbar while sparging with nitrogen. Reaction water was continuously distilled from the reaction mixture. When the refractive index reached 1.4828, the pressure was lowered to 50 mbar and the condensation reaction was continued for 2 h while glycerol was distilled from the product.
The product had a hydroxyl value of 1120 mg KOH/g, a polydispersity index of 1.39, and contained 6.9% of cyclic polyglycerols.
110.2 g of this product were reacted with 443.3 g of partially hydrogenated tallow fatty acid (C16/18) with an iodine value of 20 at a temperature of 240 °C while sparging with nitrogen. Reaction water was continuously distilled from the mixture. When the acid value reached <10 mg KOH/g, 46.5 g sebacic acid were added to the reaction mixture and the reaction was continued to an acid value of <5 mg KOH/g. Subsequently, the reaction was stopped by cooling.
Hydroxyl value: 19 mg KOH/g
Acid value: 4.5 mg KOH/g
Saponification value: 207 mg KOH/g
HLB (calculated): 3.7
Molar ratio monoacid:diacid: 7.0

*Example 5: Preparation of* [PGE 59]

500 g glycerol and 2.5 g of potassium hydroxide were heated to 240 °C at a pressure of 400 mbar while sparging with nitrogen. Reaction water was continuously distilled from the reaction mixture. When the refractive index reached 1.4828, the pressure was lowered to 50 mbar and the condensation reaction was continued for 2 h while glycerol was distilled from the product.
The product had a hydroxyl value of 1120 mg KOH/g, a polydispersity index of 1.39, and contained 6.9% of cyclic polyglycerols.
123.2 g of this product were reacted with 424.8 g of partially hydrogenated tallow fatty acid (C16/18) with an iodine value of 20 at a temperature of 240 °C while sparging with nitrogen. Reaction water was continuously distilled from the mixture. When the acid value reached <10 mg KOH/g, 51.9 g sebacic acid were added to the reaction mixture and the reaction was continued to an acid value of <3mg KOH/g. Subsequently, the reaction was stopped by cooling.
Hydroxyl value: 41 mg KOH/g
Acid value: 1.4 mg KOH/g
Saponification value: 206 mg KOH/g
HLB (calculated): 4.1
Molar ratio monoacid:diacid: 6.0

*Example 6: Preparation of* [PGE 60]

500 g glycerol and 2.5 g of potassium hydroxide were heated to 240 °C at a pressure of 400 mbar while sparging with nitrogen. Reaction water was continuously distilled from the reaction mixture. When the refractive index reached 1.4828, the pressure was lowered to 50 mbar and the condensation reaction was continued for 2 h while glycerol was distilled from the product.

The product had a hydroxyl value of 1120 mg KOH/g, a polydispersity index of 1.39, and contained 6.9% of cyclic polyglycerols

120.1 g of this product were reacted with 414.1 g of partially hydrogenated tallow fatty acid (C16/18) with an iodine value of 20 at a temperature of 240 °C while sparging with nitrogen. Reaction water was continuously distilled from the mixture. When the acid value reached <10 mg KOH/g, 65.8 g sebacic acid were added to the reaction mixture and the reaction was continued to an acid value of <3 mg KOH/g. Subsequently, the reaction was stopped by cooling.
Hydroxyl value: 24 mg KOH/g
Acid value: 1.6 mg KOH/g
Saponification value: 215 mg KOH/g
HLB (calculated): 4.0
Molar ratio monoacid:diacid: 4.6

*Example 7: Preparation of* [PGE 61]

500 g glycerol and 2.5 g of potassium hydroxide were heated to 240 °C at a pressure of 400 mbar while sparging with nitrogen. Reaction water was continuously distilled from the reaction mixture. When the refractive index reached 1.4828, the pressure was lowered to 50 mbar and the condensation reaction was continued for 2 h while glycerol was distilled from the product.

The product had a hydroxyl value of 1120 mg KOH/g, a polydispersity index of 1.39, and contained 6.9% of cyclic polyglycerols

106.3 g of this product were reacted with 366.3 g of partially hydrogenated tallow fatty acid (C16/18) with an iodine value of 20 at a temperature of 240 °C while sparging with nitrogen. Reaction water was continuously distilled from the mixture. When the acid value reached <10 mg KOH/g, 127.4 g dimer acid (Radiacid 0977, Oleon) were added to the reaction mixture and the reaction was continued to an acid value of <3 mg KOH/g. Subsequently, the reaction was stopped by cooling.
Hydroxyl value: 33 mg KOH/g
Acid value: 0.3 mg KOH/g
Saponification value: 175 mg KOH/g
HLB (calculated): 3.5
Molar ratio monoacid:diacid: 6.0

*Example Formulations:*

Hot processing was applied in all cases; all given amounts are wt.-%.

Hair Rinse Formulations

| Formulation Example # | 1 | 2 | 3 | 4 |
|---|---|---|---|---|
| TEGINACID®C, Evonik Goldschmidt GmbH (INCI: Ceteareth-25) | 0.5 | 0.5 | 0.5 | 0.5 |
| TEGO®Alkanol 16, Evonik Goldschmidt GmbH(INCI: Cetyl Alcohol)) | 4 | 4 | 4 | 4 |
| VARISOFT® 300, 30 %-ig, Evonik Goldschmidt GmbH (INCI: Cetrimonium Chloride) | 3.3 | | | |
| VARISOFT PATC, Evonik Goldschmidt (INCI: Palmitamidopropyltrimonium Chloride) | | 3.3 | | |
| VARISOFT EQ 65 Pellets, Evonik Goldschmidt (INCI: Distearoylethyl Dimonium Chloride) | | | 3.3 | |
| VARISOFT BT 85 Pellets, Evonik Goldschmidt (INCI: Behentrimonium Chloride) | | | | 3.3 |
| Demin. water | ad 100 | | | |
| Citric acid | ad pH 4.0±0.3 | | | |

(continued)

| Formulation Example # | 1 | 2 | 3 | 4 |
|---|---|---|---|---|
| Example 1 | 0.5 | 0.5 | | |
| Example 2 | | | 0.5 | 0.5 |

Body Wash Formulations

| Formulation Example # | 5 | 6 | 7 | 8 |
|---|---|---|---|---|
| TEXAPON® NSO, Cognis, 28%-ig (INCI: Sodium Laureth Sulfate) | 30 | 30 | 30 | 30 |
| TEGOSOFT® PC 31, Evonik Goldschmidt GmbH (INCI: Polyglyceryl-3 Caprate) | 0.5 | 0.5 | 0.5 | 0.5 |
| Perfume | 0.3 | 0.3 | 0.3 | 0.3 |
| Water | 54.1 | 54.1 | 54.1 | 54.1 |
| TEGOCEL® HPM 4000, Evonik Goldschmidt GmbH (INCI: Hydroxypropyl Methylcellulose) | 0.3 | 0.3 | 0.3 | 0.3 |
| REWOTERIC® AM C, Evonik Goldschmidt GmbH, 32%-ig (INCI: Sodium Cocoamphoacetate) | 10 | 10 | 10 | 10 |
| Citric acid monohydrate | 0.5 | 0.5 | 0.5 | 0.5 |
| REWODERM® LI S 80, Evonik Goldschmidt GmbH (INCI: PEG-200 Hydrogenated Glyceryl Palmate; PEG-7 Glyceryl Cocoate) | 2 | 2 | 2 | 2 |
| TEGO® Pearl N 300, Evonik Goldschmidt GmbH (INCI: Glycol Distearate; Laureth-4; Cocamidopropyl Betaine) | 2 | 2 | 2 | 2 |
| Example 2 | 0.3 | | | |
| Example 4 | | 0.3 | | |
| Example 5 | | | 0.3 | |
| Example 6 | | | | 0.3 |

Mild Shower Bath

| Formulation Example # | 9 | 10 | 11 | 12 |
|---|---|---|---|---|
| TEXAPON® NSO, Cognis, 28%-ig (INCI: Sodium Laureth Sulfate) | 27 | 27 | 27 | 27 |
| REWOPOL® SB FA 30, Evonik Goldschmidt GmbH, 40%-ig (INCI: Disodium Laureth Sulfosuccinate) | 12 | 12 | 12 | 12 |
| TEGOSOFT® LSE 65 K SOFT, Evonik Goldschmidt GmbH (INCI: Sucrose Cocoate) | 2 | 2 | 2 | 2 |
| Water | 39 | 39 | 39 | 39 |
| REWOTERIC® AM C, Evonik Goldschmidt GmbH, 32%-ig (INCI: Sodium Cocoamphoacetate) | 13 | 13 | 13 | 13 |
| Citric Acid (30% in water) | 3 | 3 | 3 | 3 |
| ANTIL® 171 Evonik Goldschmidt GmbH (INCI: PEG-18 Glyceryl Oleate/Cocoate) | 1.5 | 1.5 | 1.5 | 1.5 |
| TEGO® Pearl N 300 Evonik Goldschmidt GmbH (INCI: Glycol Distearate; Laureth-4; Cocamidopropyl Betaine) | 2 | 2 | 2 | 2 |

(continued)

| Formulation Example # | 9 | 10 | 11 | 12 |
|---|---|---|---|---|
| Example 1 | 0.3 | | | |
| Example 2 | | 0.3 | | |
| Example 3 | | | 0.3 | |
| Example 4 | | | | 0.3 |

Body Wash Formulations

| Formulation Example # | 13 | 14 | 15 | 16 |
|---|---|---|---|---|
| Water | 93.95 | 93.75 | 93.95 | 93.95 |
| Propylene Glycol | 1 | 1 | 1 | 1 |
| Citric acid monohydrate | q.s. | q.s. | q.s. | q.s. |
| TEGO® Alkanol 16, Evonik Goldschmidt GmbH (INCI: Cetyl alcohol) | 3 | 3 | 3 | 3 |
| VARISOFT® PATC, Evonik Goldschmidt GmbH (INCI: Palmitamidopropyltrimonium Chloride) | 1.75 | 1.75 | 1.75 | 1.75 |
| Example 1 | 0.3 | | | |
| Example 2 | | 0.3 | | |
| Example 5 | | | 0.3 | |
| Example 6 | | | | 0.3 |
| Perfume, preservative | q.s. | q.s. | q.s. | q.s. |

Leave-in Conditioning Mousse

| Formulation Example # | 17 | 18 | 19 | 20 |
|---|---|---|---|---|
| Example 1 | 0.3 | | | |
| Example 2 | | 0.3 | | |
| Example 5 | | | 0.3 | |
| Example 7 | | | | 0.3 |
| ABIL® B 88183, Evonik Goldschmidt GmbH (INCI: PEG/PPG-20/6 Dimethicone) | 0.4 | 0.4 | 0.4 | 0.4 |
| TAGAT® CH-40(INCI: PEG-40 Hydrogenated Castor Oil) | 0.5 | 0.5 | 0.5 | 0.5 |
| Perfume | 0.2 | 0.2 | 0.2 | 0.2 |
| TEGO® Betain 810, Evonik Goldschmidt GmbH, 38%-ig (INCI: Capryl/Capramidopropyl Betaine) | 4.2 | 4.2 | 4.2 | 4.2 |
| Water | 93.5 | 93.5 | 93.5 | 93.5 |
| Panthenol | 0.2 | 0.2 | 0.2 | 0.2 |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 0.3 | 0.3 | 0.3 | 0.3 |
| Citric Acid (30% in water) | 0.4 | 0.4 | 0.4 | 0.4 |

Creamy Shaving Foam

| Formulation Example # | Phase | 21 | 22 | 23 |
|---|---|---|---|---|
| Water | A | 50 | 50 | 50 |
| Coconut Fatty Acid | A | 1.4 | 1.4 | 1.4 |
| Monoethanolamine | A | 1.3 | 1.3 | 1.3 |
| Myristic Acid | A | 3.5 | 3.5 | 3.5 |
| TEGOSOFT® LSE 65 K Evonik Goldschmidt (INCI: Sucrose Cocoate) | B | 2 | 2 | 2 |
| Example 1 | B | 1.7 | | |
| Example 2 | B | | 1.7 | |
| Example 4 | B | | | 1.7 |
| TEGO® Betain 810 Evonik Goldschmidt (INCI: Capryl/Capramidopropyl Betaine) | C | 7.6 | 7.6 | 7.6 |
| Glycerin | C | 5 | 5 | 5 |
| Perfume | C | 0.3 | 0.3 | 0.3 |
| Water | C | 26.5 | 26.5 | 26.5 |
| TEGOCEL® HPM 50 Evonik Goldschmidt (INCI: Hydroxypropyl Methylcellulose) | C | 0.7 | 0.7 | 0.7 |

## Claims

1. Cosmetic composition containing polyglycerol partial ester having the structure of Formula (I)

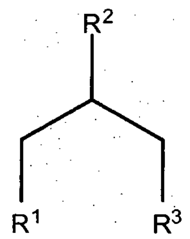

Formula (I)

with $R^1$, $R^2$ and $R^3$ independent from each other, equal or different selected from the group consisting of
-OH,
-$OR^4$, with $R^4$ a linear, unsubstituted acyl radical with a chain length of from 16 to 22 carbon atoms with the proviso that the fatty acids obtained from the acyl radical by saponification bears an iodine value of smaller than 50,
a radical having the structure of general Formula (II)

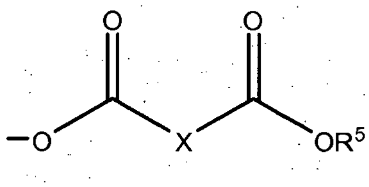

Formula (II)

with $R^5$ a radical having the structure of Formula (I) wherein one of $R^1$, $R^2$ und $R^3$ being a direct bond to the

oxygen of $-OR^5$ and with X a bivalent organic residue with from 2 to 34 carbon atoms and $-OR^5$, with $R^5$ like above

wherein each molecule of the polyglycerol partial ester comprises at least one of each $-OR^5$ and a radical having the structure of Formula (II),
with the provisos that the polyglycerol partial ester comprises an HLB-value from 2 to 10 and
that the polyglycerol obtained by hydrolysis or alcoholysis of the polyglycerol partial ester comprises an average degree of polymerization of from 2 to 8 and at least 1 % of the polyglycerol comprises cyclic structures.

**2.** Cosmetic composition according to claim 1 **characterized in that** X in the contained polyglycerol partial ester is a bivalent, linear, unsubstituted alkyl radical with 2 to 12 carbon atoms.

**3.** Cosmetic composition according to claim 1 or 2 **characterized in that** the contained polyglycerol partial ester has a molecular weight of at least 2000 g/mol.

**4.** Cosmetic composition according to at least one of the claims 1 to 3 **characterized in that** the molar ratio of monocarboxylic acid component to dicarboxylic acid component of the contained polyglycerol partial ester is 2-20.

**5.** Cosmetic composition according to at least one of the claims 1 to 4 **characterized in that** the molar ratio of the dicarboxylic acid component to polyglycerol of the contained polyglycerol partial ester is from 0.1 to 1.0.

**6.** Cosmetic composition according to at least one of the claims 1 to 5 **characterized in that** the contained polyglycerol partial ester has a melting point of at least 25 °C, preferably at least 35 °C, more preferably at least 40 °C.

**7.** Cosmetic composition according to at least one of the claims 1 to 6 **characterized in that** the contained polyglycerol partial ester is obtainable by a method comprising the steps of

1.) esterification of

a) a polyglycerol mixture comprising an average degree of polymerization of from 2 to 8, preferred from 2.5 to 6, particularly preferred from 3 to 4.5, and at least 1 % of cyclic structures, with
b) at least one monocarboxylic acid comprising a carboxylic acid $HOR^4$, with $R^4$ a linear, unsubstituted acyl radical with a chain length of from 16 to 22 carbon atoms with the proviso that the the carboxylic acid or mixture of carboxylic acids bears an iodine value of smaller than 50, preferably smaller than 30, particularly preferably smaller than 25,

2.) addition and further esterification with

c) at least one dicarboxylic acid having the structure of general Formula (IIb).

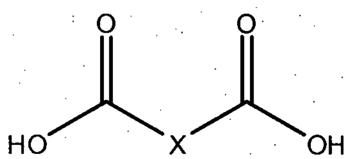

Formula (IIb)

with X a bivalent organic residue with from 2 to 34 carbon atoms or mixtures thereof

with the proviso that the ratio by weight of polyglycerol mixture to the sum of monocarboxylic acid and dicarboxylic acid is in the range from about 0.11 to 1.

**8.** Cosmetic composition according to at least one of the claims 1 to 7 **characterized in that** it is a hair care composition.

9. Use of at least one cosmetic composition according to at least one of the claims 1 to 8 or of at least one polyglycerol partial ester described in any of the claims 1 to 7 in cosmetic compositions, especially in hair care compositions.

10. Use of at least one cosmetic composition according to at least one of the claims 1 to 8 or of at least one polyglycerol partial ester described in any of the claims 1 to 7 as a conditioning agent for hair, as a hair protecting agent, as a hair repair agent or as a hair strengthening agent.

**Patentansprüche**

1. Kosmetische Zusammensetzung, enthaltend Polyglycerinpartialester mit der Struktur der Formel (I)

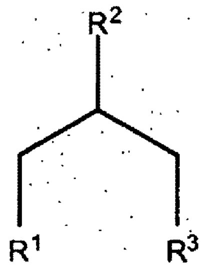

Formel (I)

wobei $R^1$, $R^2$ und $R^3$ unabhängig voneinander gleich oder verschieden sind und aus der Gruppe bestehend aus
-OH,
$-OR^4$, wobei $R^4$ für einen linearen, unsubstituierten Acylrest mit einer Kettenlänge von 16 bis 22 Kohlenstoffatomen steht, mit der Maßgabe, dass die durch Verseifung aus dem Acylrest erhaltenen Fettsäuren eine Iodzahl von weniger als 50 aufweisen,
einem Rest mit der Struktur der allgemeinen Formel (II)

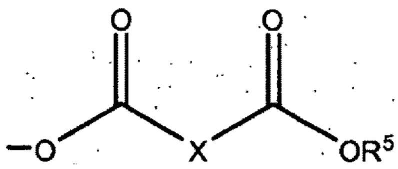

Formel (II)

wobei $R^5$ für einen Rest mit der Struktur der Formel (I) steht, worin eine der Gruppen $R^1$, $R^2$ und $R^3$ für eine direkte Bindung zu dem Sauerstoff von $-OR^5$ steht, und X für einen zweiwertigen organischen Rest mit 2 bis 34 Kohlenstoffatomen steht, und
$-OR^5$, wobei $R^5$ wie oben definiert ist,
ausgewählt sind,
wobei jedes Molekül des Polyglycerinpartialesters mindestens je eine Gruppe $-OR^5$ und einen Rest mit der Struktur der Formel (II) umfasst,
mit den Maßgaben, dass der Polyglycerinpartialester einen HLB-Wert von 2 bis 10 umfasst und
dass das durch Hydrolyse oder Alkoholyse des Polyglycerinpartialesters erhaltene Polyglycerin einen durchschnittlichen Polymerisationsgrad von 2 bis 8 umfasst und mindestens 1% des Polyglycerins cyclische Strukturen umfasst.

2. Kosmetische Zusammensetzung nach Anspruch 1, **dadurch gekennzeichnet, dass** X in dem enthaltenen Polyglycerinpartialester für einen zweiwertigen, linearen, unsubstituierten Alkylrest mit 2 bis 12 Kohlenstoffatomen steht.

3. Kosmetische Zusammensetzung nach Anspruch 1 oder 2, **dadurch gekennzeichnet, dass** der enthaltene Polyglycerinpartialester ein Molekulargewicht von mindestens 2000 g/mol aufweist.

4. Kosmetische Zusammensetzung nach mindestens einem der Ansprüche 1 bis 3, **dadurch gekennzeichnet, dass** das Molverhältnis von Monocarbonsäurekomponente zu Dicarbonsäurekomponente des enthaltenen Polyglycerinpartialesters 2-20 beträgt.

**5.** Kosmetische Zusammensetzung nach mindestens einem der Ansprüche 1 bis 4, **dadurch gekennzeichnet, dass** das Molverhältnis von Dicarbonsäurekomponente zu Polyglycerin des enthaltenen Polyglycerinpartialesters 0,1 bis 1,0 beträgt.

**6.** Kosmetische Zusammensetzung nach mindestens einem der Ansprüche 1 bis 5, **dadurch gekennzeichnet, dass** der enthaltene Polyglycerinpartialester einen Schmelzpunkt von mindestens 25°C, vorzugsweise mindestens 35°C, weiter bevorzugt mindestens 40°C, aufweist.

**7.** Kosmetische Zusammensetzung nach mindestens einem der Ansprüche 1 bis 6, **dadurch gekennzeichnet, dass** der enthaltene Polyglycerinpartialester nach einem Verfahren erhältlich ist, umfassend die Schritte

1. Veresterung von

a) einem Polyglyceringemisch mit einem durchschnittlichen Polymerisationsgrad von 2 bis 8, vorzugsweise 2,5 bis 6, besonders bevorzugt 3 bis 4,5, und mindestens 1% cyclischen Strukturen mit
b) mindestens einer Monocarbonsäure, umfassend eine Carbonsäure $HOR^4$, wobei $R^4$ für einen linearen, unsubstituierten Acylrest mit einer Kettenlänge von 16 bis 22 Kohlenstoffatomen steht, mit der Maßgabe, dass die Carbonsäure oder das Gemisch von Carbonsäuren eine Iodzahl von weniger als 50, vorzugsweise weniger als 30, besonders bevorzugt weniger als 25, aufweist,

2. Zugabe und weitere Veresterung mit

c) mindestens einer Dicarbonsäure mit der Struktur der allgemeinen Formel (IIb)

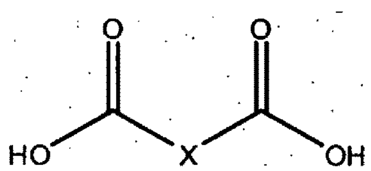
Formel (IIb)

wobei X für einen zweiwertigen organischen Rest mit 2 bis 34 Kohlenstoffatomen steht, oder Mischungen davon,

mit der Maßgabe, dass das Gewichtsverhältnis von Polyglyceringemisch zur Summe von Monocarbonsäure und Dicarbonsäure im Bereich von etwa 0,11 bis 1 liegt.

**8.** Kosmetische Zusammensetzung nach mindestens einem der Ansprüche 1 bis 7, **dadurch gekennzeichnet, dass** es sich dabei um eine Haarpflegezusammensetzung handelt.

**9.** Verwendung mindestens einer kosmetischen Zusammensetzung nach mindestens einem der Ansprüche 1 bis 8 oder mindestens eines in einem der Ansprüche 1 bis 7 beschriebenen Polyglycerinpartialesters in kosmetischen Zusammensetzungen, insbesondere in Haarpflegezusammensetzungen.

**10.** Verwendung mindestens einer kosmetischen Zusammensetzung nach mindestens einem der Ansprüche 1 bis 8 oder mindestens eines in einem der Ansprüche 1 bis 7 beschriebenen Polyglycerinpartialesters als Haarkonditionierungsmittel, als Haarschutzmittel, als Haarreparaturmittel oder als Haarstärkungsmittel.

## Revendications

**1.** Composition cosmétique contenant un ester partiel de polyglycérol répondant à la structure de Formule (I)

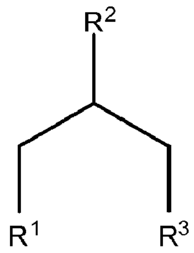 Formule (I)

$R^1$, $R^2$ et $R^3$, indépendamment les uns des autres, étant identiques ou différents et choisis dans le groupe constitué par
-OH,
$-OR^4$, $R^4$ étant un radical acyle non substitué linéaire ayant une longueur de chaîne allant de 16 à 22 atomes de carbone, à condition que les acides gras obtenus à partir du radical acyle par saponification présentent un indice d'iode inférieur à 50,
un radical répondant à la structure de Formule générale (II)

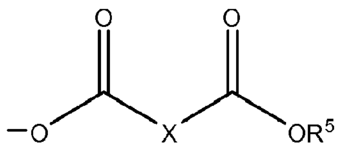 Formule (II)

$R^5$ étant un radical répondant à la structure de Formule (I), où l'un parmi $R^1$, $R^2$ et $R^3$ est une liaison directe à l'oxygène de $-OR^5$ et X étant un reste organique bivalent ayant de 2 à 34 atomes de carbone et $-OR^5$, $R^5$ étant comme ci-dessus,
où chaque molécule de l'ester partiel de polyglycérol comprend au moins un parmi chaque $-OR^5$ et un radical répondant à la structure de Formule (II),
à condition que l'ester partiel de polyglycérol possède un rapport hydro-lipophile allant de 2 à 10, et
que le polyglycérol obtenu par hydrolyse ou alcoolyse de l'ester partiel de polyglycérol comprenne un degré moyen de polymérisation allant de 2 à 8 et qu'au moins 1% du polyglycérol comprenne des structures cycliques.

**2.** Composition cosmétique selon la revendication 1, **caractérisée en ce que** X dans l'ester partiel de polyglycérol contenu est un radical alkyle bivalent, linéaire, non substitué, ayant de 2 à 12 atomes de carbone.

**3.** Composition cosmétique selon la revendication 1 ou 2, **caractérisée en ce que** l'ester partiel de polyglycérol contenu possède un poids moléculaire d'au moins 2000 g/mol.

**4.** Composition cosmétique selon au moins l'une des revendications 1 à 3, **caractérisée en ce que** le rapport molaire du composant d'acide monocarboxylique au composant d'acide dicarboxylique de l'ester partiel de polyglycérol contenu est de 2-20.

**5.** Composition cosmétique selon au moins l'une des revendications 1 à 4, **caractérisée en ce que** le rapport molaire du composant d'acide dicarboxylique au polyglycérol de l'ester partiel de polyglycérol contenu va de 0,1 à 1,0.

**6.** Composition cosmétique selon au moins l'une des revendications 1 à 5, **caractérisée en ce que** l'ester partiel de polyglycérol contenu possède un point de fusion d'au moins 25°C, préférablement d'au moins 35°C, plus préférablement d'au moins 40°C.

**7.** Composition cosmétique selon au moins l'une des revendications 1 à 6, **caractérisée en ce que** l'ester partiel de polyglycérol contenu peut être obtenu par une méthode comprenant les étapes

1) d'estérification

a) d'un mélange de polyglycérol comprenant un degré moyen de polymérisation allant de 2 à 8, préférablement de 2,5 à 6, particulièrement préférablement de 3 à 4,5, et au moins 1% de structures cycliques, avec b) au moins un acide monocarboxylique comprenant un acide carboxylique $HOR^4$, $R^4$ étant un radical acyle non substitué linéaire ayant une longueur de chaîne allant de 16 à 22 atomes de carbone, à condition que l'acide carboxylique ou le mélange d'acides carboxyliques présente un indice d'iode inférieur à 50, préférablement inférieur à 30, particulièrement préférablement inférieur à 25,

2) d'addition et d'estérification supplémentaire avec

c) au moins un acide dicarboxylique répondant à la structure de Formule générale (IIb)

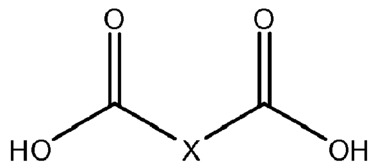
Formule (IIb)

X étant un reste organique bivalent ayant de 2 à 34 atomes de carbone,
ou des mélanges de ceux-ci,
à condition que le rapport en poids du mélange de polyglycérol à la somme de l'acide monocarboxylique et de l'acide dicarboxylique se trouve dans la plage allant d'environ 0,11 à 1.

**8.** Composition cosmétique selon au moins l'une des revendications 1 à 7, **caractérisée en ce qu'**il s'agit d'une composition de soin des cheveux.

**9.** Utilisation d'au moins une composition cosmétique selon au moins l'une des revendications 1 à 8, ou d'au moins un ester partiel de polyglycérol décrit selon l'une quelconque des revendications 1 à 7, dans des compositions cosmétiques, notamment dans des compositions de soin des cheveux.

**10.** Utilisation d'au moins une composition cosmétique selon au moins l'une des revendications 1 à 8, ou d'au moins un ester partiel de polyglycérol décrit selon l'une quelconque des revendications 1 à 7, comme agent conditionneur pour les cheveux, comme agent de protection des cheveux, comme agent de réparation des cheveux, ou comme agent de renforcement des cheveux.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- DE 3818292 A1
- EP 0451461 B1
- JP 2008308415 B
- JP 2008280329 B
- JP 2008208050 B
- JP 2008195689 B
- WO 2005115328 A
- EP 780117 A
- DE 3533600
- DE 102008001788

### Non-patent literature cited in the description

- *Fragrance Journal,* 1998, vol. 26, 64-70
- **CASSEL et al.** Original synthesis of linear, branched and cyclic oligoglycerol standards. *Eur. J. Org. Chem.,* 2001, 875-896
- The Dimer Acids: The chemical and physical properties, reactions and applications. 1975